# EUROPEAN PATENT APPLICATION

(11) **EP 4 458 374 A1**
(43) Date of publication of application: **06.11.2024**
(21) Application number: 22913248.5
(22) Date of filing: 30.06.2022
(51) Int. Cl.: A61K 39/39, A61K 47/02, A61K 45/00, A61P 37/04, A61P 35/00, B82Y 5/00, B82Y 40/00

(54) **ALUMINUM-MANGANESE COMPOSITE NANOCRYSTAL, AND PREPARATION METHOD THEREFOR AND USE THEREOF**

(30) Priority: 28.12.2021 CN 202111633153
(71) Applicant: The GBA National Institute for Nanotechnology Innovation, Guangzhou, Guangdong 510530 (CN)
(72) Inventor: CHEN, Chunying, Guangzhou, Guangdong 510530 (CN); WANG, Yaling, Guangzhou, Guangdong 510530 (CN); ZHAO, Yuliang, Guangzhou, Guangdong 510530 (CN)
(74) Representative: Günther, Constantin
(86) International application number: PCT/CN2022/102747
(87) International publication number: WO 2023/123959

(57) **Abstract**

An aluminum-manganese composite nanocrystal, and a preparation method therefor and the use thereof. The method for preparing the aluminum-manganese composite nanocrystal comprises: step 1, mixing an aluminum salt solution, a manganese salt solution and an anionic adjuvant solution to obtain a mixture, and adjusting the pH value of the mixture to 5.5-8.5; and step 2, heating the mixture for a reaction, and washing the obtained solid reactant to obtain the aluminum-manganese composite nanocrystal. According to the aluminum-manganese composite nanocrystal prepared using the preparation method and the use thereof in the preparation of a vaccine adjuvant, a pharmaceutical composition, a drug delivery carrier or an immunogenic composition, the technical problem that an existing aluminum adjuvant cannot activate humoral immunity and cell immunity at the same time can be effectively solved.

## Description

This application claims the priority of Chinese Patent Application No.202111633153.6, titled "ALUMINUM-MANGANESE COMPOSITE NANOCRYSTAL, AND PREPARATION METHOD THEREFOR AND USE THEREOF", filed on December 28, 2021 with the China National Intellectual Property Administration, which is incorporated herein by reference in its entirety.

### FIELD

This application belongs to the technical field of vaccines, and in particular relates to an aluminum-manganese composite nanocrystal, a preparation method therefor and use thereof.

### BACKGROUND

Among the existing vaccine adjuvants, aluminum salt adjuvant is the most widely used and safest immune adjuvant, including potassium aluminum sulfate, aluminum phosphate and aluminum hydroxide. Among them, aluminum hydroxide adjuvant is the most widely used. At present, the mechanism of aluminum adjuvant stimulating immunity is mainly explained by "reservoir" effect, phagocytosis promotion and activation of NLRP3, a pro-inflammatory pathway. Commercial aluminum hydroxide adjuvant is usually boehmite with weak crystal form, with a size of 1 µm to 10 µm. It generally activates humoral immunity and rarely T cell-mediated immunity, which is related to the way that aluminum hydroxide adjuvant absorbs antigen and presents it. When micron-sized aluminum hydroxide adjuvant adsorbs antigen, aluminum adjuvant generally binds to the cell membrane of antigen presenting cells, such as dendritic cells (DC cells), inducing the formation of lipid vesicles, activating downstream pathways, and failing to interfere with the fate of presenting antigen in cells. The antigen is processed through the lysosomal pathway and presented through the major histocompatibility complex II (MHC-II), rather than through the cross-presentation of the major histocompatibility complex I (MHC-I). However, the latest research shows that in order to meet the needs of more universal diseases, not only humoral immunity is needed to produce antibodies to neutralize pathogenic microorganisms, but also T cells are needed to mediate immunity to remove viruses, bacteria or abnormal physiological reactions, and memory T cells are produced to prevent and protect the body. Therefore, we need to find a new aluminum adjuvant formula or composite adjuvant to meet the needs of preventive vaccine protection.

Because of the advantages of long-term safety and convenience of aluminum adjuvant, and its convenience as antigen and molecular adjuvant carrier, the new adjuvant formed by compounding aluminum salt and molecular adjuvant has gradually become the main trend of new adjuvant development. For example, the composite adjuvant formed by aluminum salt and 3M-052, CpG and TLR agonists has shown better advantages in improving antibody titer of vaccine and cellular immunity ability. However, the pre-preparation of molecular adjuvants and the process of compounding with aluminum adjuvants have many steps, which can easily affect the effectiveness of new adjuvants. Therefore, it will be more practical to develop a new composite adjuvant that has the characteristics of convenient preparation of aluminum adjuvant, sustained release of carrier and antigen and high safety, and can make up for the deficiency of aluminum adjuvant in cellular immunity, while being easy to prepare and produce.

### SUMMARY

In view of this, the present application provides an aluminum-manganese composite nanocrystal, a preparation method therefor and use thereof, which can effectively solve the technical problem that existing aluminum adjuvant cannot activate humoral immunity and cellular immunity at the same time.

In a first aspect, the present application provides a method for preparing an aluminum-manganese composite nanocrystal, comprising:
Step 1, mixing an aluminum salt solution, a manganese salt solution and an anionic auxiliary agent solution to obtain a mixture, and adjusting a pH of the mixture to 5.5 to 8.5;
Step 2, heating the mixture for a reaction, and washing the obtained solid reactant to prepare the aluminum-manganese composite nanocrystal.

Specifically, in step 1, an acid-base regulator is adopted to adjust the pH of the mixture, and the acid-base regulator can be an alkali solution or/and an acid solution, the alkali solution and the acid solution are existing conventional solutions for adjusting the pH, and the alkali solution has a concentration of 0.01 mol/L to 1 mol/L, preferably 0.05 mol/L to 0.6 mol/L. The alkali solution can be one or more of sodium hydroxide aqueous solution, potassium hydroxide aqueous solution, ammonia water, sodium bicarbonate aqueous solution and sodium phosphate aqueous solution. The sodium hydroxide aqueous solution has a concentration of 0.05 mol/L to 1 mol/L, and preferably the sodium hydroxide aqueous solution has a concentration of 0.3 mol/L to 0.6 mol/L.

Specifically, the pH of the mixture is adjusted, and the aluminum-manganese composite nanocrystal obtained within this range is stable and will not be changed after sterilization.

Specifically, the phosphate of the sodium phosphate aqueous solution is selected from Na2HPO4 and/or Na3PO4.

Specifically, in step 1, the molar ratio of the sum of the solutes of the aluminum salt solution, the manganese salt solution and the anionic auxiliary agent solution to the solute of the alkali solution is 1:(1-6), for example, the molar ratio of the sum of the solutes of the aluminum salt solution, the manganese salt solution and the anionic auxiliary agent solution to NaOH of the alkali solution is 1:(1-6); preferably, the molar ratio of the sum of the solutes of the aluminum salt solution, the manganese salt solution and the anionic auxiliary agent solution to the solute of the alkali solution is 1:(2-4), for example, the molar ratio of the sum of the solutes of the aluminum salt solution, the manganese salt solution and the anionic auxiliary agent solution to NaOH of the alkali solution is 1:(2-4).

Specifically, in step 1, the mixture has a pH value of 5.5 to 8.0, and preferably, the mixture has a pH value of 6.0 to 7.0.

Specifically, in step 1, the aluminum salt solution, the manganese salt solution and the anionic auxiliary agent solution are mixed by a mixing method of stirring while adding the solution dropwise. The aluminum salt solution, the manganese salt solution and the anionic auxiliary agent solution are added dropwise in a speed of 2 mL/min to 15 mL/min, preferably 3 mL/min to 8 mL/min, and stirred in a speed of 50 rpm to 1000 rpm, preferably 300 rpm to 800 rpm during mixing, for a time of 1.5 h to 10 h, preferably 1 h to 5 h.

Specifically, when an alkali solution is used to adjust the pH value of the mixture, the alkali solution is added dropwise in a speed of 0.5 mL/min to 10 mL/min, preferably 1 mL/min to 4 mL/min.

Specifically, in step 2, centrifugal washing is performed using NaCl solution to remove unreacted free ions and desorbed anionic auxiliary agent, and finally redissolution and dispersion are performed using NaCl solution. The obtained mixture is sterilized by moist heat at 121 °C for 30 min to 60 min, and packaged to obtain aluminum-manganese composite nanocrystals. The aluminum-manganese composite nanocrystals can be stored in cold storage (2 °C to 8 °C). The centrifugal washing is performed in a centrifugal rotational speed of 2000 g to 10000 g, for a centrifugal time of 1 min to 100 min and washing times of 2 to 5 times; preferably, the centrifugal washing is performed in a centrifugal rotational speed of 4000 g to 8000 g, for a centrifugal time of 5 min to 20 min and washing times of 2 to 3 times.

Specifically, the NaCl solution has a concentration of 0.1% to 1.5%, and preferably, the NaCl solution has a concentration of 0.15% to 0.8%.

In another embodiment, a solute of the aluminum salt solution is selected from the group consisting of aluminum chloride, aluminum sulfate, aluminum nitrate, aluminum acetate, and a mixture thereof; a solvent of the aluminum salt solution is selected from the group consisting of sodium acetate solution, physiological saline, water, ethanol, and a mixture thereof. Specifically, the solvent of the aluminum salt solution has a concentration of 0.01 mol/L to 1 mol/L, for example, the sodium acetate solution has a concentration of 0.03 mol/L to 0.07 mol/L.

In another embodiment, a solute of the manganese salt solution is selected from the group consisting of manganese chloride, manganese sulfate, manganese nitrate, manganese acetate, and a mixture thereof; a solvent of the manganese salt solution is selected from the group consisting of water, physiological saline, ethanol, and a mixture thereof.

In another embodiment, a solute of the anionic auxiliary agent solution is selected from organic acid salts and/or amino acids; a solvent of the anionic auxiliary agent solution is selected from the group consisting of water, physiological saline, ethanol, and a mixture thereof.

Specifically, the anionic auxiliary agent solution can fully complete the coordination between anionic auxiliary agent and metal ions (aluminum and manganese), and the pre-precipitate in the mixture is heated to promote the uniform growth of the pre-precipitate in the mixture, promote the formation of crystals, and improve the sterilization stability. The mixture is subjected to centrifugal precipitation, washing and sterilization to obtain aluminum-manganese composite nanocrystals.

Specifically, the anionic auxiliary agent is an organic substance containing polyhydroxy, carboxyl structure and phosphorus-containing elements. The anionic auxiliary agent of this application can form a relatively stable complex with aluminum ions and manganese ions, and is an ideal auxiliary agent for stabilizing and regulating the particle size and distribution of aluminum-manganese composite nanocrystals.

In another embodiment, the organic acid salt is selected from citric acid and/or salicylic acid; the amino acid is selected from the group consisting of the cysteine, cystine, tyrosine, aspartic acid, glutamic acid, and a mixture thereof.

In another embodiment, in the mixture, a molar ratio of aluminum element in the aluminum salt solution to manganese element in the manganese salt solution is 1: (0.05-1); preferably, the a molar ratio of aluminum element in the aluminum salt solution to manganese element in the manganese salt solution is 1: (0.1-0.5). A ratio of the concentration of the anionic auxiliary agent to a total concentration of metal ions is (0.1-10): 1, preferably, a ratio of the concentration of the anionic auxiliary agent to a total concentration of metal ions is (0.3-3): 1. The total concentration of the metal ion is a sum of the concentrations of the aluminum salt solution and the manganese salt solution.

Specifically, the aluminum salt solution has a concentration of 0.01 mol/L to 1 mol/L, preferably 0.03 mol/L to 0.07 mol/L; the manganese salt solution has a concentration of 0.001 mol/L to 1 mol/L, preferably 0.003 mol/L to 0.05 mol/L; the anionic auxiliary agent solution has a concentration of 0.01 mol/L to 0.5 mol/L, and preferably 0.05 mol/L to 0.5 mol/L.

In another embodiment, in step 2, the heating for a reaction is performed at 60 °C to 130 °C for a time of 0.5 h to 20 h.

In a second aspect, the present application provides an aluminum-manganese composite nanocrystal, comprising the aluminum-manganese composite nanocrystal prepared by the preparation method.

Specifically, the pH value of the aluminum-manganese composite nanocrystal before and after sterilization is 5.5 to 8.5. The aluminum-manganese composite nanocrystal has a grain size of 20 nm to 5000 nm, and the grain size remains stable. The adsorption rate of the aluminum-manganese composite nanocrystal on bovine serum albumin is more than 1.6 mg, and the obtained aluminum-manganese composite nanocrystal product has excellent dispersibility and stability.

In a third aspect, the present application provides use of the aluminum-manganese composite nanocrystal prepared by the preparation method and the aluminum-manganese composite nanocrystal in preparing a vaccine adjuvants, an immune enhancer, a pharmaceutical composition, a drug delivery carrier or an immunogenic composition.

Specifically, the present application provides use of the aluminum-manganese composite nanocrystals in preparing a vaccine adjuvant and an immune enhancer for improving innate immunity and/or adaptive immunity. In some embodiments, the aluminum-manganese composite nanocrystals improve innate immunity and/or adaptive immunity and regulate immune balance by stimulating the expression of type I interferon and γ interferon. In still other embodiments, the aluminum-manganese composite nanocrystals can promote antigen presentation and enhance antibody production level to improve innate immunity and/or adaptive immunity. In still other embodiments, the aluminum-manganese composite nanocrystals promote the activation of multiple immune pathways to improve innate immunity and/or adaptive immunity by joint, combination or sequential administration with immunomodulators. The aluminum-manganese composite nanocrystal of the present application can be used as an immune enhancer, which be used for tumor prevention and/or treatment of diseases, such as bacterial infection, fungal infection, viral infection, parasitic infection, tumor and autoimmune disease.

In addition, the aluminum-manganese composite nanocrystals of the present application can be used as a pharmaceutical composition to treat autoimmune diseases, wherein the autoimmune diseases are selected from rheumatoid arthritis, type I diabetes, psoriasis, systemic lupus erythematosus and multiple sclerosis. More specifically, when the aluminum-manganese composite nanocrystal of the present application is used as a component of a pharmaceutical composition, the pharmaceutical composition comprises the aluminum-manganese composite nanocrystal and a preventive/therapeutic agent, both of which are administered together to a subject in need. It can be combined with immunomodulators, antibody drugs and polypeptide drugs such as small molecules, polypeptides, antibodies through blending, sequential administration and modification.

Specifically, the drug delivery carrier comprises a composition in which aluminum-manganese nanocrystals are combined with liquid, solid or common pharmaceutical adjuvants and/or optional one or more adjuvant components. Pharmaceutical adjuvants are pharmaceutically acceptable emulsions, microemulsions, solutions, suspensions, syrups, inert diluents, wetting agents, emulsifiers and dispersants.

In a fourth aspect, the present application provides an anti-tumor pharmaceutical composition, comprising the aluminum-manganese composite nanocrystal and a drug for treating anti-tumor diseases.

Specifically, the present application provides use of the anti-tumor pharmaceutical composition in treating solid tumors and non-solid tumors; the solid tumor is lung cancer, gastric cancer, breast cancer, liver cancer, pancreatic cancer, skin cancer, oral cancer, esophageal cancer, malignant melanoma, head and neck cancer, sarcoma, cholangiocarcinoma, bladder cancer, renal cancer, colorectal cancer, placental choriocarcinoma, cervical cancer, ovarian cancer, testicular cancer, uterine cancer and leukemia and the like; the non-solid tumor is glioma, haematoma and the like.

Specifically, the drugs for treating anti-tumor diseases are anti-tumor small molecules, polypeptides and antibody drugs, such as one or more of PD-L1 antibody, trastuzumab, metformin (Met), paclitaxel, adriamycin, cisplatin, asparaginase, rituximab, gefitinib, triptorelin, docetaxel, doxorubicin and fluorouracil.

In a fifth aspect, the present application provides a vaccine composition, comprising an antigen and the aluminum-manganese composite nanocrystal.

Specifically, the vaccine composition is a protein vaccine or/and an inactivated vaccine, such as influenza vaccine strain recombinant antigen and hepatitis B recombinant antigen; the vaccine composition can also be varicella zoster virus recombinant protein vaccine, novel coronavirus subunit protein vaccine or novel coronavirus inactivated vaccine.

Specifically, the antigens in the vaccine composition are derived from viruses, bacteria, parasites and tumor neoantigens. For example, the viruses are selected from DNA virus and RNA virus, preferably, the virus is selected from Coronaviridae, Herpesviridae, Rhabdoviridae, Filoviridae, Orthomyxoviridae, Paramyxovirus, Picornaviridae, Hepadnaviridae, Flaviviridae, Papillomavirus, Poxviridae, and Retroviridae, and more preferably, the virus is selected from novel coronavirus, influenza virus, herpes simplex virus, vesicular stomatitis virus, vaccinia virus, HIV or HBV For example, the bacteria are selected from Gram-positive bacteria and Gram-negative bacteria, and preferably, the bacteria are selected from Streptococcus pneumoniae, Haemophilus influenzae, Salmonella, Neisseria meningitidis, Staphylococcus epidermidis, Staphylococcus aureus, Escherichia coli, Klebsiella pneumoniae, Klebsiella oxytoca, Enterobacter cloacae, Citrobacter freundii, Pseudomonas aeruginosa, Acinetobacter baumannii, Mycobacterium tuberculosis or Helicobacter pylori.

More specifically, the antigen in the vaccine composition is the antigen derived from influenza virus, α coronavirus or β coronavirus (such as novel coronavirus and variant strains), hepatitis virus (such as hepatitis A virus and hepatitis B virus), poliovirus, rabies virus, HPV virus, encephalitis virus (such as encephalitis B virus), mumps virus, rubella virus, tetanus bacillus, pertussis bacillus, diphtheria bacillus, leprosy bacillus, mycobacterium tuberculosis, meningococcus, pneumococci and any combination thereof in inactivated virus/bacterial form, attenuated live virus/bacterial form, and subunit protein, recombinant protein or chimeric protein, and the like.

Specifically, the water used in this application can be ultrapure water or/and injection water.

Manganese is an essential trace element in the process of human growth, metabolism, neuron function and antioxidant defense. Combining two inorganic elements, aluminum and manganese, to form a composite adjuvant can not only retain the "reservoir" effect of aluminum adjuvant itself, promote phagocytosis and activate the pro-inflammatory pathway NLRP3, but also supplement the inherent immunity and adaptive immunity mediated by Mn2+, so as to meet the requirements of humoral immunity and cellular immunity against infectious pathogens at the same time. Meanwhile, the preparation method provided by the present application can prepare aluminum-manganese composite nanocrystals with uniform size and controllable morphology.

Therefore, the present application aims to disclose an aluminum-manganese composite nanocrystal which is simple in process, uniform in composition, stable in structure, long-term storable after sterilization and convenient to use and can be used as a vaccine adjuvant. The aluminum-manganese composite nanocrystal can activate humoral immunity and cellular immunity at the same time, and provide a better immune barrier for the prevention of infectious bacteria or viruses.

### BRIEF DESCRIPTION OF DRAWINGS

FIG 1 is a transmission electron microscope diagram of aluminum-manganese composite nanocrystal (AlMn-001) provided in Example 1 of this application;
FIG 2 is a transmission electron microscope diagram of aluminum-manganese composite nanocrystal (AlMn-002) provided in Example 2 of this application;
FIG 3 is a transmission electron microscope diagram of aluminum-manganese composite nanocrystal (AlMn-003) provided in Example 3 of this application;
FIG 4 is the XRD results of aluminum-manganese composite nanocrystals provided in Examples 1 to 3 of this application;
FIG 5 is the DC activation effect of aluminum-manganese composite nanocrystals with different proportions of aluminum and manganese provided in Examples 1 to 3 of the present application;
FIG 6 is a fluorescence imaging image of endocytosis of DC cells after aluminum-manganese composite nanocrystal (AlMn-003) provided in Example 3 of this application or commercial aluminum adjuvant is combined with antigens;
FIG 7 is the result of antibody titer detection after intramuscular injection of mice with aluminum-manganese composite nanocrystals (AlMn-003) provided in Example 3 of the present application or commercial aluminum adjuvant in combination with OVA antigen;
FIG 8 shows the result of antibody titer in mice after immunization with the aluminum-manganese composite nanocrystals provided in Examples 1 to 3 of this application in combination with novel coronavirus RBD recombinant protein antigen;
FIG 9 is the result of antibody titer detection after intramuscular injection of mice with the aluminum-manganese composite nanocrystal (AlMn-003) provided in Example 3 of this application or commercial aluminum adjuvant Alum in combination with H1N1 HA antigen;
FIG 10 is the result of IFN-γ expression in the spleen cells of mice 56 days after immunization with the aluminum-manganese composite nanocrystal AlMn-003 provided in Example 3 of this application in combination with H1N1 HA antigen;
FIG 11 is the result of cytokines in the spleen cells of mice 56 days after immunization with the aluminum-manganese composite nanocrystal AlMn-003 provided in Example 3 of this application in combination with H1N1 HA antigen;
FIG 12 is the proportion of memory B cells in the spleen and lungs of mice 56 days after immunization with the aluminum-manganese composite nanocrystal AlMn-003 provided in Example 3 of this application in combination with H1N1 HA antigen;
FIG 13 is the proportion of memory T cells in the spleen of mice 56 days after immunization with the aluminum-manganese composite nanocrystal AlMn-003 provided in Example 3 of this application in combination with H1N1 HA antigen;
FIG 14 is the effect of the aluminum-manganese composite nanocrystal AlMn-001 provided in Example 1 of the present application as an immune synergist for enhancing the anti-tumor of the body, and the three diagrams from left to right are FIG 14A, FIG 14B and FIG 14C respectively;
FIG 15 is the effect of the aluminum-manganese composite nanocrystal AlMn-002 provided in Example 2 of the present application as an immune synergist for enhancing the anti-distal tumor of the body;
FIG 16 is the effect of the aluminum-manganese composite nanocrystal AlMn-003 provided in Example 3 of the present application as an immune synergist for enhancing the anti-liver cancer of the body;
FIG 17 is the effect of the aluminum-manganese composite nanocrystal AlMn-001 provided in Example 1 of the present application as an immune synergist for enhancing the anti-metastatic tumor of the body.

### DETAILED DESCRIPTION

The present application provides an aluminum-manganese composite nanocrystal and a preparation method therefor and use thereof, for solving the technical defect that the existing aluminum adjuvant in the prior art cannot activate humoral immunity and cellular immunity at the same time.

Technical solutions of examples of the present application are clearly and completely described below. Apparently, the examples described are only some examples of the present application, rather than all the examples. All the other examples obtained by those skilled in the art based on the examples in the present application without any creative work belong to the protection scope of the present application.

The raw materials or reagents used in the following examples are all commercially available or self-made.

### Example 1

An aluminum-manganese composite nanocrystal AlMn-001 is provided in the example of the present application. Its specific preparation method comprises:
(1) AlCl₃•6H₂O was weighed and dissolved in 0.01 mol/L NaAc to prepare an aluminum salt solution with an aluminum ion content of 1 mol/L. MnCl₂•4H₂O was weighed and dissolved in water for injection to prepare a manganese salt solution with a manganese ion content of 0.1 mol/L. Salicylic acid was weighed and dissolved in pure water to prepare 0.05 mol/L anionic auxiliary agent solution. NaOH was weighed and dissolved with water for injection to prepare an alkali solution with a NaOH content of 0.1 mol/L.
(2) First, the aluminum salt solution and the manganese salt solution were mixed in equal volume to obtain a mixed solution. 30 mL of the mixed solution was taken out and added to 50 mL of the salicylic acid solution at a speed of 10 mL/ min, and stirred at a speed of 1000 rpm for 3 hours, so that the coordination reaction fully occurred to obtain a mixture.
(3) At room temperature, the rotation speed was adjusted to 600 rpm and the mixture was stirred at this rotation speed. 30 mL of ammonia water solution was added at a speed of 12 mL/ min. After the dropwise addition, the pH value of the mixture was adjusted to 7.6, and the temperature was raised to 100 °C, and the mixture was reacted for 1 hour.
(4) The obtained mixture was cooled to room temperature, and centrifuged in a centrifuge at the rotation speed of 6000 g for 20 minutes, and the obtained solid reactant was washed twice with 0.5% sodium chloride solution. Then it was centrifuged at 6000 g for 10 minutes and redissolved with 0.5% sodium chloride solution to obtain the aluminum-manganese composite nanocrystal, which was labeled AlMn-001. Then, it was sterilized by moist heat at 121 °C for 30 minutes and then packaged.
(5) The aluminum-manganese composite nanocrystal was stored in cold storage for a long time (2 °C to 8 °C).

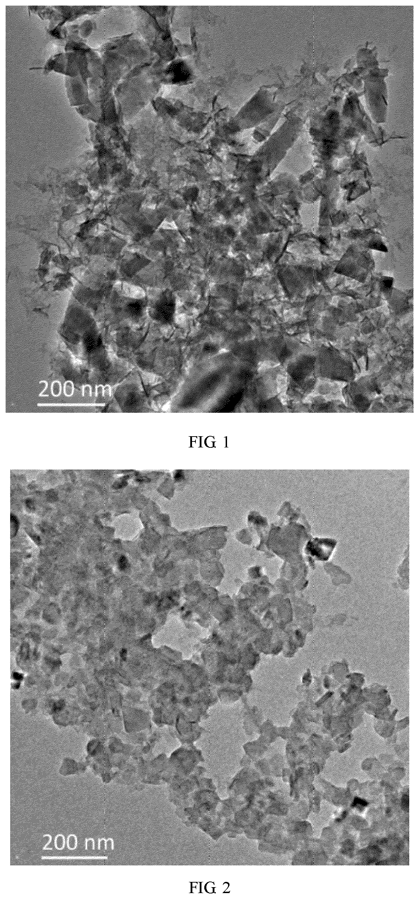

Reference is made to FIG 1. FIG 1 is an image of the size and morphology of the aluminum-manganese composite nanocrystal prepared in Example 1 of this application measured by an electron transmission microscope (FEI company, model Tecnai G2 20S-TWIN). As can be seen from FIG 1, the aluminum-manganese composite nanocrystal prepared in Example 1 of this application is composite particles stacked with flaky nanosheets with a width of 30 nm to 120 nm and a length of 50 nm to 300 nm.

### Example 2

An aluminum-manganese composite nanocrystal AlMn-002 is provided in the example of the present application. Its specific preparation method comprises:
Al(NO₃)₃•6H₂O was weighed and dissolved in 0.01 mol/L NaAc to prepare an aluminum salt solution with an aluminum ion content of 0.4 mol/L. Mn(SO₄)₂•4H₂O was weighed and dissolved in water for injection to prepare a manganese salt solution with a manganese ion content of 0.1 mol/L. Glutamate is weighed and dissolved in pure water solution to prepare an anionic auxiliary agent solution with a concentration of 0.20 mol/L. KOH was weighed and dissolved with water for injection to prepare an alkali solution with a KOH content of 0.5 mol/L.

(2) First, the aluminum salt solution and the manganese salt solution were mixed in equal volume to obtain a mixed solution, 40 mL of the mixed solution was taken out and added into a container containing 30 mL of glutamic acid aqueous solution, and which was reacted at a stirring speed of 800 rpm for 7 hours at room temperature, so that the coordination reaction was fully occurred to obtain a mixture.

(3) The rotation speed was adjusted to 500 rpm and the mixture was stirred at this rotation speed. Then potassium hydroxide solution was added at the speed of 6.9 mL/ min to make the pH of the solution 8.5, the temperature was adjusted to 80 °C, and the mixture was stably reacted for 7 hours.

(4) The obtained mixture was cooled to room temperature, and centrifuged in a centrifuge at the rotation speed of 6000 g for 20 minutes, and the obtained solid reactant was washed twice with 0.5% sodium chloride solution. Then it was centrifuged at 6000 g for 10 minutes and redissolved with 0.5% sodium chloride solution to obtain the aluminum-manganese composite nanocrystal, which was labeled AlMn-002.

(5) Then, it was sterilized by moist heat at 121 °C for 30 minutes and then packaged. The aluminum-manganese composite nanocrystal was stored in cold storage for a long time (2 °C to 8 °C).

Reference is made to FIG 2. FIG 2 is an image of the size and morphology of the aluminum-manganese composite nanocrystal prepared in Example 2 of this application measured by an electron transmission microscope (FEI company, model Tecnai G2 20S-TWIN). As can be seen from FIG 2, the aluminum-manganese composite nanocrystal prepared in Example 2 of this application is composite particles stacked with flaky nanosheets with a width of 20 nm to70 nm and a length of 30 nm to 100 nm.

### Example 3

An aluminum-manganese composite nanocrystal AlMn-003 is provided in the example of the present application. Its specific preparation method comprises:
(1) Aluminum acetate was weighed and dissolved in 0.01 mol/L NaAC to prepare an aluminum salt solution with an aluminum ion content of 0.5 mol/l. MnCl₂•4H₂O was weighed and dissolved in water for injection to prepare a manganese salt solution with a manganese ion content of 0.1875 mol/L. Citric acid was taken out and prepared into an anionic auxiliary agent solution with a concentration of 0.1 mol/L. Sodium hydroxide was weighed and dissolved with water for injection to prepare an alkali solution with a content of 0.2 mol/L.
(2) First, the aluminum salt solution and the manganese salt solution were mixed in equal volume to obtain a mixed solution, and 10mL of the mixed solution was added into a reactor containing 50mL of citric acid solution, and the mixture was stirred at 1200 rpm for 1 hour at room temperature, so that the coordination reaction was fully occurred to obtain a mixture.
(3) Then, 10 mL of aqueous sodium hydroxide solution was added dropwise at the speed of 16.9 mL/ min. The pH of the solution was adjusted to 9, the temperature was raised to 60 °C, and the mixture was stably reacted for 12 hours.
(4) The obtained mixture was cooled to room temperature, and centrifuged in a centrifuge at the speed of 8000 g for 10 minutes, and the obtained solid reactant was washed three times with 0.5% sodium chloride solution. Then it was centrifuged at 8000 g for 10 minutes and redissolved with 0.5% sodium chloride solution to obtain the aluminum-manganese composite nanocrystal, which was labeled AlMn-003.
(5) Then, it was sterilized by moist heat at 121 °C for 30 minutes and then packaged. The aluminum-manganese composite nanocrystal was stored in cold storage for a long time (2 °C to 8 °C).

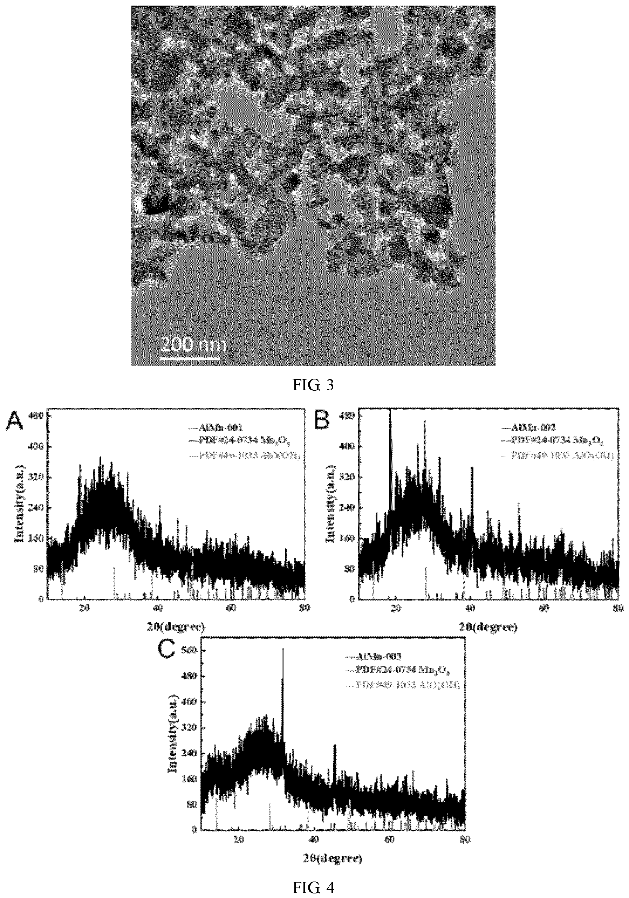

Reference is made to FIG 3. FIG 3 is an image of the size and morphology of the aluminum-manganese composite nanocrystal prepared in Example 3 of this application measured by an electron transmission microscope (FEI company, model Tecnai G2 20S-TWIN). As can be seen from FIG 3, the aluminum-manganese composite nanocrystal prepared in Example 3 of this application is composite particles stacked with flaky nanosheets with a width of 20 nm to 100 nm and a length of 30 nm to 200 nm.

### Example 4

The physical and chemical properties test of the aluminum-manganese composite nanocrystals in Examples 1 to 3 are provided in the example of the present application, specifically including:
The following test methods are suitable for determining the physical and chemical properties of any kind of aluminum-manganese composite nanocrystal with the molar ratio of Mn: Al.

### (1) Characterization of hydrated particle size and potential:

The aluminum-manganese composite nanocrystals prepared in Example 1, Example 2 and Example 3 were diluted by 100 to 200 times with ultra-pure water until they were clear and colorless, and 800 µL of the mixture was taken out to Zeta potential cell for testing.

### (2) X-ray diffraction (XRD) characterization:

The aluminum-manganese composite nanocrystals were put into a centrifuge tube, and freeze-dried, and the solid powder was uniformly filled into the window of the glass sample table, and then compacted with a glass slide, and the convex part was scraped flat, so that the powder was full of the whole concave window and was flush with the upper surface of the glass slide. The sample table was loaded into the X-ray diffractometer, in which the parameters were set in a software, and then scanned.

The results are shown in FIG 4. As can be seen from FIG 4, all manganese contained in the aluminum-manganese composite nanocrystal samples obtained in Examples 1 to 3 of this application is a crystal form of trimanganese tetroxide, and all aluminum is a crystal form of AlO(OH).

### (3) Digestion quantification of aluminum-manganese composite nanocrystals:

The aluminum-manganese composite nanocrystals obtained in Examples 1 to 3 of this application were diluted by 10 times with ultrapure water, and 100 µL of the mixture was put into a 50 mL polytetrafluoroethylene digestion bottle, and 2 mL to 3 mL of concentrated nitric acid of MOS grade was added. The mixture was heated to a temperature of 90 °C on an electric heating plate, and 2 mL of hydrogen peroxide of MOS grade was added dropwise to make the solution a state of clear and transparent. Then the solution was gradiently heated to a temperature of 160 °C, in which the temperature was increased by 10 °C each time and stayed for 20 minutes, and slowly evaporated until the solution remained 0.5 mL, and then removed. The obtained mixture was cooled to room temperature, and adjusted the volume to 3 g. 3% washing solution was prepared with nitric acid of MOS grade, and standard curve was prepared with a standard sample. The obtained mixture was detected and quantified by inductively coupled plasma mass spectrometry.

(4) Adsorption rate: the adsorption rate was calculated based on the adsorption of bovine serum albumin by the aluminum-manganese composite nanocrystal.

### Preparation of test sample solution:

A proper amount of test sample was taken out, and diluted with 0.85% to 0.90% sodium chloride solution until the aluminum content was 1 mg/mL, and the pH value was adjusted to 6.0 to 7.0 to obtain the test sample solution. The pH value of different batches of the same aluminum-manganese adjuvant should be adjusted to a fixed value to ensure the consistency of different batches.

### Bovine serum albumin (abbreviated as bovine albumin) solution:

A proper amount of bovine serum albumin was taken out. A 10 mg/mL solution was prepared with 0.85% to 0.90% sodium chloride solution, and the pH value was adjusted to be consistent with that of the test solution.

### Determination method:

Five 15 ml centrifuge tubes ware taken out, and 0.08 ml, 0.16 mL, 0.4 mL, 0.8 mL and 1.2 mL of bovine serum albumin solution (10 mg/ml) were added into the centrifuge tubes respectively, and 0.85% to 0.90% sodium chloride solution was added to adjust the final volume of the mixture to 4.0 mL. After uniformly evenly, 1.0 mL of the test solution was added to each tube respectively, uniformly mixed, so that the amount of bovine serum albumin in each tube was 0.8 mg, 1.6 mg, 4 mg, 8 mg and 12 mg respectively, and each tube contained 1 mg of aluminum.

Each of the above tubes was placed at room temperature for 1 hour (during which the tubes were vigorously shaken once every 10 minutes), and centrifuged at a speed of 5000 g for 10 minutes. The supernatant was collected. The content of free bovine serum albumin in the supernatant of each tube was measured by Lowry method (the second method of General Rule 0731) or other appropriate methods. The absorbance value of each tube was recorded and the protein content was calculated. The adsorption rate (A) of each tube was calculated according to the total amount of bovine serum albumin corresponding to the free bovine serum albumin content in the supernatant of each tube. The volume of supernatant was calculated as 5ml. Adsorption rate = (total bovine albumin content - supernatant bovine albumin content) ÷ total bovine albumin content ×100.

### Results determination:

The protein in the supernatant of the 2 tubes with bovine albumin content of 0.8 mg and 1.6 mg should not be detected, that is, the adsorption rate should not be lower than 90%. Moreover, the absorbance value of each tube with bovine albumin content of 4 mg, 8 mg and 12 mg should show an overall increasing trend (if the absorbance value cannot be determined, the overall increasing trend should be determined by detecting the protein content in the supernatant). The adsorption rate is judged as qualified.

### (5) 24-hour sedimentation rate:

The pH value of the test sample was adjusted to 6.0 to 7.0 with dilute hydrochloric acid or sodium hydroxide solution. The test sample was diluted to 5 mg/mL of aluminum with water. If the aluminum content of the test sample was lower than 5 mg/mL, it was diluted to 1 mg/mL with 9 g/L sodium chloride solution after adjusting the pH. If the aluminum content of the test sample was less than 1 mg/mL, it was let stand for a suitable time, and the supernatant was discarded to make the aluminum content reach 1 mg/mL. The sample was shaken for at least 30 seconds, and 25 mL of the solution was taken into a measuring cylinder or graduated colorimetric tube, and let stand for 24 hours. According to the amount of separated supernatant, the sedimentation rate of the sample was calculated according to the following formula: $sedimentation rate \left(%\right) = supernatant volume / 25 \times 100 %$

In the above examples, the hydrated particle size and surface charge of aluminum hydroxide adjuvant were measured by a nano-particle size and Zeta potential analyzer (purchased from Malvern Company, Zetasizer Nano ZS model). The aluminum content and manganese content of aluminum-manganese composite adjuvant were measured by inductively coupled plasma atomic emission spectrometer (ICP-OES, purchased from Perkin Elmer Company, PerkinElmer Optima 5300DV model). The adsorption degree and sedimentation were determined with reference to the Chinese Pharmacopoeia (2020 edition). The above test results are shown in Table 1.

**Table 1**

| Test Items | AlMn-001 of Example 1 | AlMn-002 of Example 2 | AlMn-003 of Example 3 |
|---|---|---|---|
| Average particle size (µm) | 2.39 | 1.73 | 2.15 |
| Aluminum content (mg/mL) | 0.89 | 0.88 | 0.61 |
| Manganese content (mg/mL) | 0.10 | 0.24 | 0.33 |
| Potential (mV) | +26 | +20 | +15 |
| Adsorption capacity (mg) | >1.6 | > 1.6 | > 1.6 |
| Settlement rate% | 15% to 20% | 15% to 20% | 15% to 20% |

### Example 5

This example of the present application provides comparison of BMDC activation ability of the aluminum-manganese composite nanocrystals prepared in Examples 1 to 3, including:

BMDC activation effect of the aluminum-manganese composite nanocrystal AlMn-001, the aluminum-manganese composite nanocrystal AlMn-002 and the aluminum-manganese composite nanocrystal AlMn-003 prepared in Examples 1 to 3 of this application was evaluated.

Immature BMDC was inoculated into a 6-well plate with 5×10⁵ cells per well. After adherence, 100 µl of commercial aluminum adjuvant Alum with a total metal ion concentration of 50 µg/ml (purchased from Invivogen Company, CAS:21645-51-2), aluminum-manganese composite nanocrystal AlMn-001 obtained in Example 1, aluminum-manganese composite nanocrystal AlMn-002 obtained in Example 2, and aluminum-manganese composite nanocrystal AlMn-003 obtained in Example 3 were added to each well, three replicates in each group. After incubation for 24 hours, the cells were collected and stained with anti-CD11c, anti-CD80 and anti-CD86 flow staining solutions. The expression levels of costimulatory factors CD80 and CD86 on the surface of BMDCs were determined by flow cytometry.

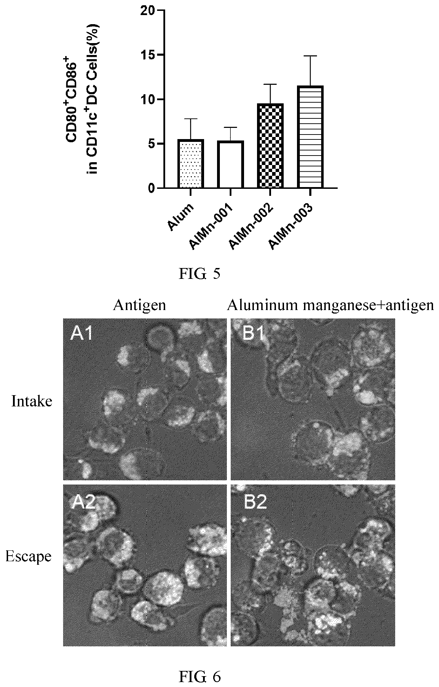

As can be seen from FIG 5, the activation ability of aluminum-manganese composite nanocrystals obtained in the examples of this invention on dendritic cells increased with the increase of Mn ratio, and the dendritic activation ability of the aluminum-manganese composite nanocrystal AlMn-003 on dendritic cells was about 2 times that of commercial aluminum adjuvant. This indicates that the aluminum-manganese composite nanocrystals obtained in this application have a more excellent dendritic cell activation ability.

### Example 6

This example of the present application provides cell uptake effects of aluminum-manganese composite nanocrystals after adsorbing antigens, including:

DC2.4 cells were cultured in RPMI 1640 medium containing 10% (v/v) FBS in a humid environment of 37°C and 5% CO₂. The cells were incubated with the constructed nanovaccine (a mixed solution of FITC-labeled antigen molecules with a concentration of 10 mg/L and aluminum-manganese composite nanocrystal AlMn-003 with a concentration of 25 mg/L or 100 mg/L of commercial aluminum adjuvant Alum (purchased from Invivogen Company, CAS: 21645-51-2) Alum) for 8 h and 24 h, respectively. Then, the cells were washed and labeled with Lysotracker Red, fixed and observed by fluorescence microscope (Perkin Elmer Spinning Disc Confocal Microscope, 60× oil immersion microscope), and images were obtained by using Ultra VIEW VoX software.

As shown in FIG 6, FIG 6 is an endocytosis fluorescence imaging image of DC cells after the aluminum-manganese composite nanocrystal AlMn-003 provided in Example 3 of this application or aluminum adjuvant Alum binds to antigens.

The experiment results are shown in FIG 6. The aluminum-manganese composite nanocrystal obtained in Example 3 of this application can still carry more antigens into cells than commercial aluminum adjuvant Alum, even when the dosage is only one quarter of that of commercial aluminum adjuvant. This shows that the aluminum-manganese composite nanocrystals obtained in this application have a more excellent vaccine carrying effect.

### Example 7

This example of the present application provides verification of the dose-effect relationship of the immune enhancement effect of the aluminum-manganese composite nanocrystals in Examples 1 to 3, specifically including:

The molar ratio of Mn:Al in the aluminum-manganese composite nanocrystals in Examples 1 to 3 is different. The aluminum-manganese composite nanocrystals with different molar ratios of Mn/Al were combined with OVA antigens and injected into mice by intramuscular injection, respectively, to determine the titers of the produced specific antibody. The specific method is as follows:
Experimental animals: Balb/C mice, 6 to 8 weeks old, 5 in each group, female.
Control group: commercial aluminum adjuvant Alum (purchased from Invivogen Company, CAS: 21645-51-2).
Dosage: OVA antigen (Ovalbumin OVA was purchased from Shanghai Yuanye Biotechnology Co., Ltd., CAS: 9006-59-1): 10 µg/mice; aluminum-manganese composite adjuvant: 50 µg/100 µL/mice; aluminum adjuvant: 50 µg/100 µL/ mice.
Experimental group: (1) AlMn-001 (Mn/Al = 0.1); (2)AlMn-002 (Mn/Al = 0.25); (3) AlMn-003 (Mn/Al = 0.375).
Immunization scheme: All drugs mentioned above were mixed with antigen in equal volume, and injected intramuscularly at intervals of 3 weeks. Mice were immunized with 3 injections. The mice were took blood from their orbits at 19 d, 35 d and 56 d after immunization in groups. The antibody titers in serum were determined by ELISA.

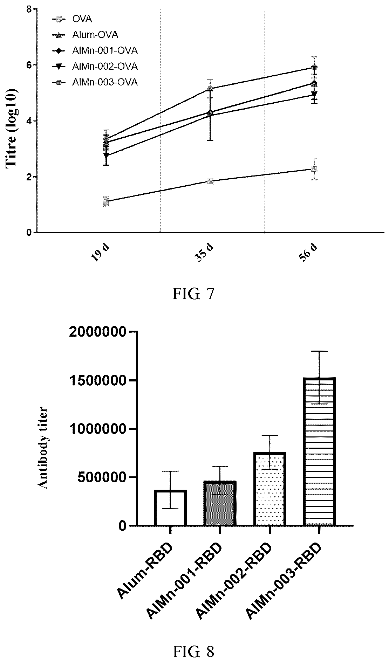

The experimental results are shown in Figure 7. In the aluminum-manganese composite nanocrystalline groups with different molar ratios of Mn/Al in Examples 1 to 3, mice not only took effect quickly after one injection of immunization, but also had an obvious positive dose-effect relationship. After 2 injections of immunization, the dose-effect relationship was still obvious, indicating that aluminum-manganese composite nanocrystals have a significant effect in enhancing the humoral immunity of OVA vaccine.

### Example 8

This example of the present application provides an experiment on enhancing the immune protection effect tests of the aluminum-manganese composite nanocrystals of Examples 1 to 3 on the SARS-CoV-2 RBD recombinant subunit protein antigen, specifically including:
Experimental animals: Balb/C mice, 6 to 8 weeks old, 5 in each group, female.
Dosage: antigen 10 µg/mice; Commercial aluminum adjuvant Alum (purchased from Invivogen Company, CAS: 21645-51-2): 100 µg/100 µl/mice; AlMn-003 aluminum-manganese composite nanocrystal: 100 µg /100 µl/ mice;
Experimental group: (1) novel coronavirus spike protein RBD (abbreviated as RBD antigen, purchased from Sino Biological, article number 40592-V08H4); (2) commercial aluminum adjuvant Alum+RBD; (3) aluminum-manganese composite nanocrystals obtained in Examples 1 to 3 +RBD, namely AlMn-001+ RBD, AlMn-002+ RBD and AlMn-003+ RBD;
Immunization scheme: The mice were immunized with group (1). In the other two groups, mice were immunized with mixture of equal volumes of commercial aluminum adjuvant Alum or aluminum-manganese composite nanocrystal AlMn-003 obtained in Example 3 and antigen. Mice were first immunized according to immunization groups, and then boosted in the third week. Blood was collected from the orbits 14 days after the second immunization to determine the antibody titer in serum.

The experimental results are shown in FIG 8. The antibody titer still increases with the increase of manganese proportion in the aluminum-manganese composite nanocrystals, and is higher than the antibody titer of the commercial aluminum adjuvant. The aluminum-manganese composite nanocrystals also have a good immune enhancement ability against novel coronavirus subunit protein antigen.

### Example 9

This example of the present application provides an experiment on the protective effect of the aluminum-manganese composite nanocrystals in Example 3 on the influenza subunit vaccine, specifically including:
The prepared aluminum-manganese composite nanocrystal AlMn-003 was used as adjuvant, which was intramuscularly injected into mice in combination with influenza H1N1 HA antigen. The titers of the produced specific IgG and IgM antibodies were determined. The detailed method is as follows:
Experimental animals: Balb/C mice, 6 to 8 weeks old, 10 in each group, female.
Dosage: H1N1 HA (purchased from Sino Biological, article number 40731-V07H) antigen 5 µg/ mice; Commercial aluminum adjuvant Alum (purchased from Invivogen Company, CAS: 21645-51-2): 100 µg/100 µl/mice; AlMn-003 aluminum-manganese composite nanocrystal: 100 µg/100 µl/ mice;
Experimental group: (1) H1N1 HA antigen; (2) Commercial aluminum adjuvant Alum+H1N1 HA; (3) aluminum-manganese composite nanocrystal AlMn-003 obtained in Example 3 + H1N1 HA;
Immunization scheme: The mice were immunized with group (1). In the other two groups, mice were immunized with mixture of equal volumes of commercial aluminum adjuvant Alum or aluminum-manganese composite nanocrystal AlMn-003 obtained in Example 3 and antigen. Mice were first immunized according to immunization groups, and then boosted in the third week. Blood was collected from the orbits two weeks after the second immunization to determine the antibody titer in serum.

The antibody titers in serum were detected by enzyme-linked immune sorbent assay (ELISA), to evaluate the vaccine-induced IgG and IgM levels in serum of mice. In brief, 96-well microtiter plates were pre-coated with H1N1 HA respectively, incubated overnight at 4 °C, and sealed with 2% BSA at 37 °C for 2 hours. Then serially diluted mice serum was added to a 96-well plate, then incubated at 37 °C for 1 hour, and then washed with PBS four times. The bound antibody reacted with HRP-conjugated goat anti-mice IgG at 37 °C for 1 hour. After washing with PBS for four times, the substrate 3,3',5,5'- tetramethylbenzidine (TMB; Sigma) was added to the plate, and 0.05% H₂SO₄ was added to terminate the reaction. The absorbance at 450 nm and 630 nm was measured in an ELISA plate reader (Tecan, San Jose, CA).

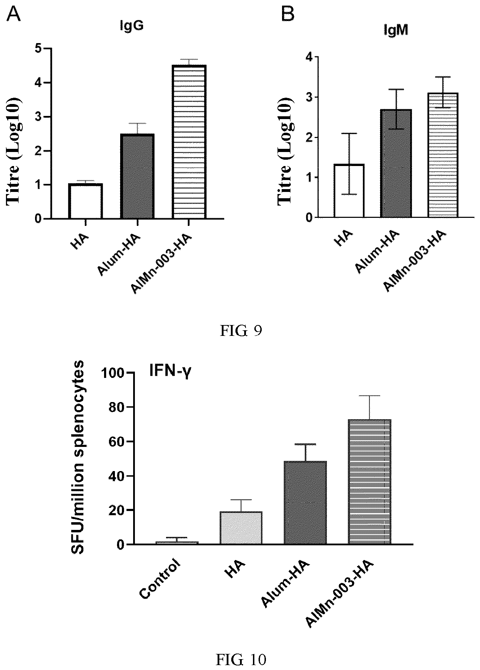

FIG 9 shows the analysis results of specific antibody titers produced 3 weeks after intramuscular injection of mice with the aluminum-manganese composite nanocrystal AlMn-003 provided in Example 3 of this application or commercial aluminum adjuvant Alum in combination with H1N1 HA antigen and 2 weeks after the second immunization.

The experimental results are shown in FIG 9A. Compared with the commercial aluminum adjuvant, the aluminum-manganese composite nanocrystal obtained in Example 3 of the present application has a better immune response, and the produced antibody titer is 104 times that of the aluminum adjuvant.

The experimental results are shown in FIG 9B. Compared with the commercial aluminum adjuvant, the aluminum-manganese composite nanocrystal obtained in Example 3 of the present invention has a higher IgM level, and the antibody titer is three times that of the aluminum adjuvant.

The tests used in subsequent Examples 10 to 13 were derived from mice immunized with different vaccines in Example 9. 56 days after immunization, five of them were euthanized. Spleen and lung tissues were collected to prepare cell suspensions for subsequent tests to evaluate the immune efficacy of the vaccines.

### Example 10

This example of the present application provides T cell test for detecting antigen-specific IFN-γ by ELISPOT after mice were immunized with aluminum-manganese composite nanocrystal of Example 3 and commercial aluminum adjuvant Alum, specifically including:
1) ELISPOT plates were pre-coated with anti-IFN-γ antibody (5 µ5/ml) and incubated at 4 °C overnight.
2) The RPMI 1640 medium containing 10% FBS was sealed at room temperature for 2 h.
3) The spleen of mice immunized in Example 9 for 56 days was ground with 200 mesh gauze to prepare a single cell suspension, with each well added with 5×10⁵ cells, and incubated in an incubator with 5% CO₂ at 37 °C for 24 h.
4) Biotinylated anti-IFN-γ antibody was added and incubated at room temperature for 2 h.
5) HRP cross-linked avidin was added and incubated at room temperature for 1 h.
6) The plate was read using Immunospot Analyzer Elispot Reader, and the number of cells secreting IFN-γ (IFN-yspot-forming cells, SFC) was read by Immunospot software (v.3.0).

The experimental results are shown in FIG 10. AlMn-003 aluminum-manganese composite nanocrystal promotes T cells to secrete γ-IFN, and the amount of γ-IFN secretion is greater than that of commercial aluminum adjuvant Alum. The commercial aluminum adjuvant Alum group promotes T cells to secrete γ-IFN+, which is 43.5% of the PBMC in the H1N1 HA antigen group, and the AlMn-003 aluminum-manganese composite nanocrystal group is 73% of the H1N1 HA antigen group, indicating that AlMn-003 aluminum-manganese composite nanocrystals have a better effect in stimulating cellular immune response.

### Example 11

This example of the present application provides an experiment on intracellular factor determination performed on spleen cells extracted from spleen tissues of mice 56 days after immunization with different vaccines collected in Example 9, specifically including:

Flow cytometry assay was performed by multicolor intracellular cytokine staining determination, using 2 µg/ml protein (H1N1 HA protein) to stimulate mouse-derived spleen cells. DMSO was used as a negative control (background). In the presence of Golgistop (BD Biosciences) and Brefeldin A (Sigma), 10 million cells were incubated at 37°C and 5% CO₂ for 6 hours. Intracellular cytokine staining assays were performed using DAPI-live/dead viability dye (Invitrogen) and anti-CD3, anti-CD4, anti-CD8, anti-CD69, anti-CD134, and anti-CD137 antibodies to assess cell viability (Biolegend) for surface staining. Cells were permeabilized and stained with anti-IL-2, anti-IL-4, anti-IFN-γ and anti-TNF-α antibodies (Biolegend). At least 100,000 to 200,000 lymphocytes were collected by BD LSR Fortessa (BD Biosciences) and analyzed using FlowJo software (Tree Star, Ashland, OR). Samples were defined as positive if the antigen-specific reaction was at least three times larger than the background and at least 0.05% higher than the background.

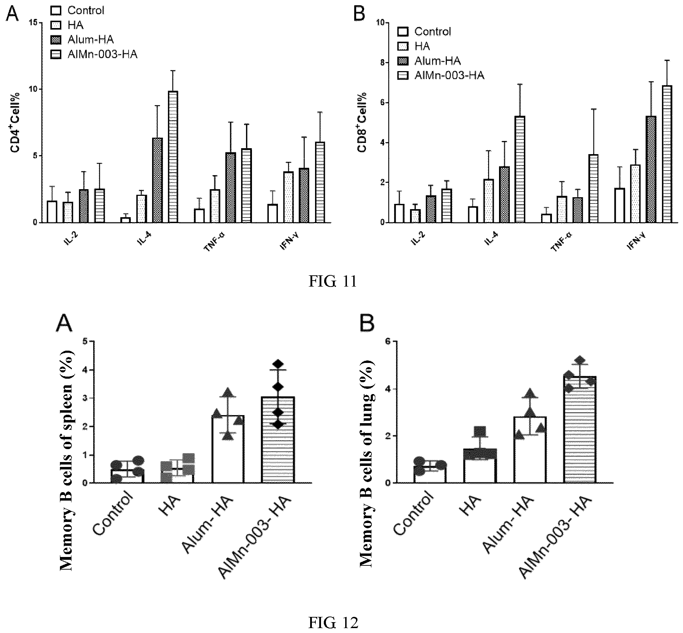

As shown in FIG 11, the levels of IL-2, IL-4, TNF-α and IFN-γ in the cytokines in the spleen are all higher, and the level of IL-4 is higher than that of IL-2. IL-2 belongs to Th1 type factor and IL-4 belongs to Th2 type factor. This indicates that the aluminum-manganese composite nanocrystal AlMn-003 of this application can promote the secretion of IFN-γ factor, IFN-γ factor can enhance the phagocytosis of macrophages, and the aluminum-manganese composite nanocrystal AlMn-003 can also promote the differentiation of T cells and the maturation of CD8 CTL.

### Example 12

This example of the present application provides an experiment on the level of memory B cells in the spleen and lung of mice 56 days after immunization with different vaccines in Example 9, specifically including:

B cells can produce high affinity antibodies, generate immune memories, act as antigen presenting cells, and secrete cytokines (including CCL22, CCL17, IL-2, IL-4, IL-6, IFN-γ, TNF-α, GM-CSF, IL-10, TGF-β1, IL-35). Memory B cells and plasma B cells can generate antibodies such as immunoglobulin (Ig), IgM, IgG and IgE. Memory B cells have a very long life span and remain dormant until they encounter antigens again. When exposed to antigens again, memory B cells can immediately generate antibodies against their antigen. With re-exposure to exogenous antigens, memory B cells are immediately reactivated and differentiated into plasma cells that can secrete antibodies. IgG isotype antibodies are secreted by these plasma cells and have high affinity for specific exogenous antigens. These IgG antibodies can effectively and quickly neutralize virus and bacterial antigens, and are part of the secondary immune response including immune memory, especially from memory B cells and memory T lymphocytes.

Therefore, this application evaluated the memory B cell level of mice 56 days after immunization with the aluminum-manganese composite nanocrystal AlMn-003 adjuvant of Example 3 in combination with H1N1 HA antigen.

The memory B cell level of mice 56 days after immunization with the aluminum-manganese composite nanocrystal AlMn-003 of Example 3 in combination with H1N1 HA antigen was measured, and the results are shown in FIG 12. According to the results of memory B cell flow cytometry, the aluminum-manganese composite nanocrystal group can induce more memory B cells in spleen and lung, and has good potential to resist secondary infection.

### Example 13

This example of the present application provides an experiment on the level of memory T cells in the spleen of mice 56 days after immunization with different vaccines in Example 9, specifically including:
Considering that memory CD4+ T cells and CD8+ T cells are the key components of protective immunity, inducing effective memory T cell response is the main goal of chronic infection vaccine. The present application examined the phenotype of memory T cells of mice on the 56^{th} day after H1N1 HA vaccine immunization.

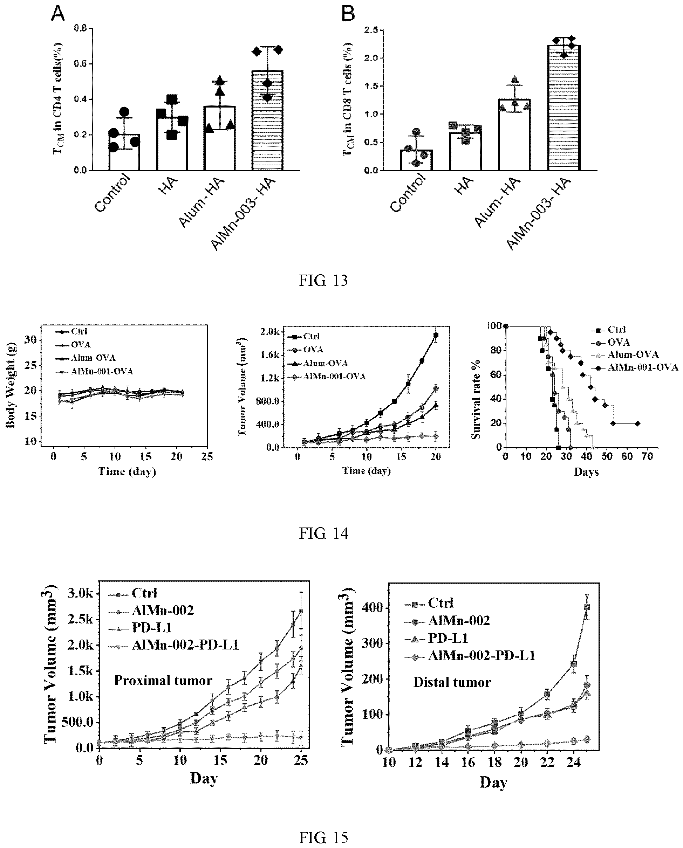

The CD4+ T and CD8+ T cell levels of mice 56 days after immunization with the aluminum-manganese composite nanocrystal AlMn-003 in Example 3 in combination with H1N1 HA antigen were measured. The experimental results are shown in Figure 13. The results of flow cytometry show that CD4+T and CD8+ T cells in spleen show the increase of central memory T cells (Tcm) in the aluminum-manganese composite nanocrystal group.

### Example 14

This example of the present application provides anti-tumor effect of the aluminum-manganese composite nanocrystal AlMn-001 of Example 1 as an immunopotentiator, specifically including:
Mice were randomly divided into four groups according to their body weight, namely: blank control group (Ctrl), OVA control group, OVA and AlMn-001 (AlMn-001-OVA), and OVA and commercial aluminum adjuvant group (Alum-OVA). According to the experimental design, mice were immunized for three times (once every 7 days), and then melanoma B16-OVA was inoculated subcutaneously to establish an in situ tumor model. As shown in FIGs. 14A to 14C, FIG 14A shows the change in body weight of mice in each group, FIG 14B shows the change in tumor volume of mice in each group, and FIG 14C shows the survival rate of mice in each group. As shown in FIG 14A, the body weight of mice in each group did not change significantly. As shown in FIG 14B, at the 20^{th} day, the average tumor size of mice in the control group was 1982 mm³. The commercial aluminum adjuvant immunization group can inhibit the growth of tumor to a certain extent, and the average tumor size was 734.9 mm³, whereas the AlMn-001 of this application could effectively inhibit the growth of tumor, and the average tumor size of immunized mice was 201.7 mm³. As shown in FIG 14C, the longest survival time of the control mice was 26 days, and the longest survival time of the mice immunized with commercial aluminum adjuvant was 43 days, whereas some mice in the AlMn-001 immunization group still survived at the 65^{th} day, and the survival time of other mice in the group was also significantly prolonged. The results show that the pre-use of aluminum adjuvant could only slightly inhibit tumor growth and improve the survival rate of mice, whereas the pre-use of AlMn-001 could significantly inhibit tumor growth, improve the survival rate of mice and prolong the survival time. AlMn-001 can be used as a tumor immunopotentiator to enhance the body's resistance to in situ tumors.

### Example 15

This example of the present application provides an anti-breast cancer distal tumor test of the aluminum-manganese composite nanocrystal AlMn-002 in Example 2 in combination with PD-L1 antibody, specifically including:
100 µl of 4T1 cells (1×10⁵) were inoculated into the right hind leg of Balb/c mice, which was recorded as an in situ tumor. When the average tumor volume reached 100 m³, mice were randomly divided into four groups: control group (Ctrl), AlMn-002 group, PD-L1 antibody group (purchased from Biolegend Company, USA, catalog: 124329, clone 10F.9G2), AlMn-002-PD-L1 antibody combination group. The tail vein was injected with materials (ALMN-002 100µg/ time/mice). It was recorded as day 0. The mice in each group were treated accordingly on day 1, day 3, and day 5 respectively. The tumor volume of mice in each group was recorded every two days. On the 10^{th} day, 5×10⁴ 4T1 cells were inoculated on the left hind leg of mice, which were recorded as distal tumors. The tumor volumes on both sides were recorded until the 25^{th} day.

The results are shown in FIG 15, which shows the therapeutic effect of aluminum-manganese composite nanocrystal AlMn-002 in combination with PD-L1 antibody on distal tumors of breast cancer. As can be seen from FIG 15, the combination of aluminum-manganese composite nanocrystal AlMn-002 and PD-L1 antibody can not only significantly inhibit the growth of existing tumors, but also significantly inhibit the growth of new tumors.

### Example 16

This example of the present application provides anti-liver cancer tumor test of the aluminum-manganese composite nanocrystal AlMn-003 in Example 3 in combination with metformin (Met), specifically including:
100 µl of HepG2 cells (1×10⁵) were inoculated into the right hind leg of Balb/c mice. When the average tumor volume reached 100 mm³, the mice were randomly divided into four groups: control group (Ctrl), AlMn-003 group, metformin (Met, purchased from Dalian Meilun Bio, CAS: 657-24-9) group, AlMn-003-Met combined group. The tail vein was injected with materials (ALMN-003 100µm/time/mice). It was recorded as the day 0, and the mice in each group were treated accordingly on day 1, day 3, and day 5 respectively. The tumor volume of mice in each group was recorded every two days.

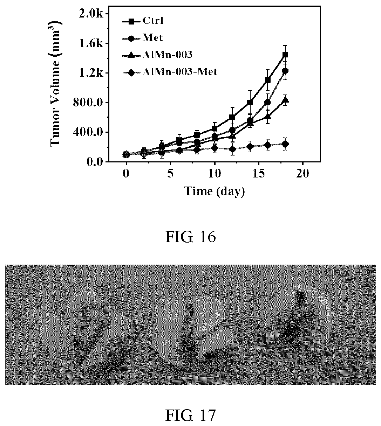

The results are shown in FIG 16. FIG 16 shows the therapeutic effect of aluminum-manganese composite nanocrystal AlMn-003 in combination with Met antibody on liver cancer tumor model. As can be seen from FIG 16, the combination of aluminum-manganese composite nanocrystal AlMn-003 and Met antibody has a significant inhibitory effect on the growth of liver cancer model tumor.

### Example 17

This example of the present application provides an experiment of inhibiting the growth of metastatic tumors by the aluminum-manganese composite nanocrystal AlMn-001 of Example 1, specifically including:
A 4T1 breast cancer model in mice was established. The tumor-bearing mice were randomly divided into three groups, and injected intratumorally with (1) normal saline, (2) paclitaxel (purchased from Shanghai Acmec Biochemical Technology Co., Ltd., CAS: 33069-62-4) and (3)AlMn-001- paclitaxel respectively. The mice were treated once every 2 days for 3 times. On the 10^{th} day, 100 µL PBS containing 4×10⁵ 4T1 cells was injected into the tail vein for induction of lung metastasis. On the 28^{th} day, the mice were killed, and the lung tissue was removed. The results are shown in FIG 17, which shows the effect of AlMn-001 in combination with paclitaxel in the treatment of liver cancer metastasis (the leftmost is the blank control group, the middle is the group treated with paclitaxel alone, and the rightmost is the group treated with AlMn-001 combined with paclitaxel). The results show that the lungs of mice in the blank control group were full of metastatic tumors, and the lungs of the mice treated with paclitaxel alone had a certain number of metastatic tumors, whereas the lungs of the mice treated with AlMn-001 had almost no tumors. The results show that AlMn-001 can be used as an immunopotentiator to enhance the body's anti-metastatic effect.

To sum up, the aluminum-manganese composite nanocrystals of the present application have the same ease of use as commercial aluminum adjuvants, and can have better DC cell activation effect, better antigen presentation effect, and better effects of promoting antigen cell uptake and lysosomal escape at a lower concentration than commercial aluminum adjuvant, and can activate humoral immunity and cellular immunity at the same time. Furthermore, it also has a strong tumor immunotherapy enhancement ability, and can be used as a delivery system of combined immunotherapy agents to enhance the immunotherapy effect.

The above are only preferred embodiments of the present invention. It should be pointed out that those skilled in the art can make some improvements and modifications without departing from the principles of the present invention, and these improvements and modifications should also be regarded as the protection scope of the present invention.

## Claims

1. A method for preparing an aluminum-manganese composite nanocrystal, comprising:
step 1, mixing an aluminum salt solution, a manganese salt solution and an anionic auxiliary agent solution to obtain a mixture, and adjusting a pH of the mixture to 5.5 to 8.5;
step 2, heating the mixture for a reaction, and washing the obtained solid reactant to prepare the aluminum-manganese composite nanocrystal.

2. The method according to claim 1, wherein a solute of the aluminum salt solution is selected from the group consisting of aluminum chloride, aluminum sulfate, aluminum nitrate, aluminum acetate, and a mixture thereof; a solvent of the aluminum salt solution is selected from the group consisting of sodium acetate solution, physiological saline, water, ethanol, and a mixture thereof;
a solute of the manganese salt solution is selected from the group consisting of manganese chloride, manganese sulfate, manganese nitrate, manganese acetate, and a mixture thereof; a solvent of the manganese salt solution is selected from the group consisting of water, physiological saline, ethanol, and a mixture thereof.

3. The method according to claim 1, wherein a solute of the anionic auxiliary agent solution is selected from organic acid salts and/or amino acids; a solvent of the anionic auxiliary agent solution is selected from the group consisting of water, physiological saline, ethanol, and a mixture thereof.

4. The method according to claim 3, wherein the organic acid salt is selected from citric acid and/or salicylic acid; the amino acid is selected from the group consisting of cysteine, cystine, tyrosine, aspartic acid, glutamic acid, and a mixture thereof.

5. The method according to claim 1, wherein in the mixture, a molar ratio of aluminum element in the aluminum salt solution to manganese element in the manganese salt solution is 1: (0.05-1); a ratio of the concentration of the anionic auxiliary agent to a total concentration of metal ions is (0.1-10): 1; the total concentration of the metal ion is a sum of the concentrations of the aluminum salt solution and the manganese salt solution.

6. The method according to claim 1, wherein in the step 2, the heating for a reaction is performed at 60 °C to 130 °C for a time of 0.5 h to 20 h.

7. An aluminum-manganese composite nanocrystal comprising the aluminum-manganese composite nanocrystal prepared by the method according to any one of claims 1 to 6.

8. Use of the aluminum-manganese composite nanocrystal prepared by the method according to any one of claims 1 to 6 and the aluminum-manganese composite nanocrystal according to claim 7 in preparing a vaccine adjuvant, an immune enhancer, a pharmaceutical composition, a drug delivery carrier or an immunogenic composition.

9. An anti-tumor pharmaceutical composition comprising the aluminum-manganese composite nanocrystal according to claim 7 and a drug for treating anti-tumor diseases.

10. A vaccine composition comprising an antigen and the aluminum-manganese composite nanocrystal according to claim 7.
